(19) Europäisches Patentamt
European Patent Office
Office européen des brevets

(11) Numéro de publication: **0 249 556 B1**

(12) **FASCICULE DE BREVET EUROPEEN**

(45) Date de publication du fascicule du brevet: **29.08.90**

(51) Int. Cl.⁵: **C07C 69/96**, C07C 68/02

(21) Numéro de dépôt: **87401306.3**

(22) Date de dépôt: **11.06.87**

(54) **Procédé de préparation d'esters organiques alpha-halogénés de l'acide carbonique.**

(30) Priorité: **13.06.86 FR 8608537**

(43) Date de publication de la demande: **16.12.87 Bulletin 87/51**

(45) Mention de la délivrance du brevet: **29.08.90 Bulletin 90/35**

(84) Etats contractants désignés: **CH DE GB IT LI SE**

(56) Documents cités:
**EP-A- 0 040 153**
**EP-A- 0 136 214**

(73) Titulaire: **SOCIETE NATIONALE DES POUDRES ET EXPLOSIFS, 12, quai Henri IV, F-75181 Paris Cédex 04(FR)**

(72) Inventeur: **Senet, Jean-Pierre, 79, rue de la Gare Herbeauvilliers-Buthiers, F-77760 La Chapelle La Reine(FR)**
Inventeur: **Sennyey, Gérard, 1, place des 4 Pavés Saint-Aubin, F-91190 Gif-Sur-Yvette(FR)**
Inventeur: **Wooden, Gary, Bâtiment 7C Résidence de Bel Air, F-91540 Mennecy(FR)**

**EP 0 249 556 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

L'invention concerne un nouveau procédé de préparation de carbonates alpha-halogénés de formule :

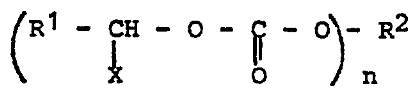

Les carbonates alpha-halogénés sont très utiles comme intermédiaires de synthèse de produits pharmaceutiques ou de produits insecticides.

La préparation des carbonates alpha-halogénés a auparavant été effectuée par halogénation radicalaire des carbonates comme décrit dans le brevet allemand DE nº 208 347 :

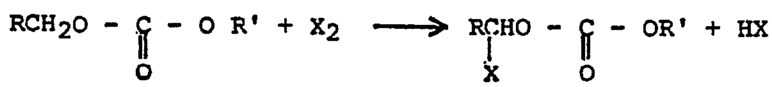

L'halogène que l'on fixe, est alors souvent le chlore ou le brome.

Mais cette halogénation présente des inconvénients. Les réactions sont longues et la mise en oeuvre des lampes UV-visibles nécessite beaucoup d'énergie. Des sous-produits halogénés sont toujours obtenus et en quantité assez importante, par conséquent seuls les carbonates dont la séparation est aisée, peuvent être préparés. De plus lorsque les carbonates sont porteurs de liaisons carbone-carbone insaturées ou de fonctions réactives il est impossible de les halogéner par ce procédé sans transformer en même temps ces liaisons ou fonctions.

Un autre procédé consiste à faire réagir des halogénoformiates alpha-halogénés avec des alcools ou des phénols :

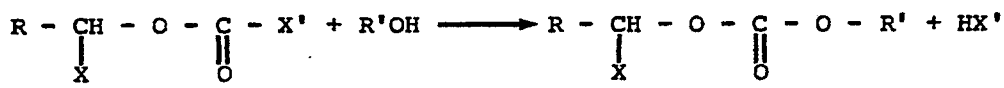

Mais d'une part certains alcools n'existent pas et la synthèse des halogénoformiates alpha-halogénés de départ n'est pas toujours facile. D'autre part dans la plupart des cas il est nécessaire d'utiliser un accepteur d'acide, comme mentionné dans les brevets DE nº2 311 328 et EP nº 108 547.

Il est également possible d'obtenir les carbonates alpha-halogénés par échange d'halogènes à partir d'un carbonate alpha-halogéné, le plus souvent alpha-chloré, et d'un halogènure de métal alcalin :

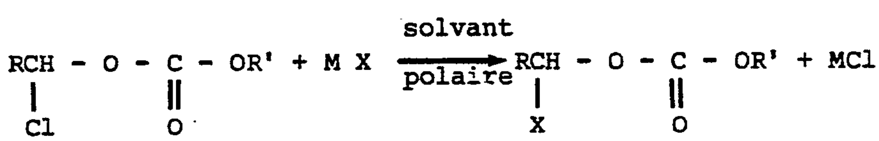


X représente alors le brome ou l'iode et M un métal alcalin, comme décrit dans les brevets FR nº 2 532 933, EP nº 108 547 et DE nº 2 816 873.

Mais pour mettre en oeuvre ce procédé il est nécessaire de préparer préalablement les carbonates alpha-chlorés par les méthodes précédentes dont nous avons montré les difficultés. La réaction d'échange chlore-brome ou chlore-iode qui est effectuée ensuite est lente et incomplète. L'isolement et la purification des carbonates alpha-iodés est difficile ou même impossible car la plupart du temps ils se décomposent dans les conditions de cette réaction.

On a par ailleurs fait réagir des aldéhydes avec du phosgène ou du chloroformiate de trichlorométhyle en présence de catalyseurs tels que des amines tertiaires, des urées substituées, des halogénures d'onium et des halogénures métalliques associés à un complexant de leur cation, comme décrit dans les brevets EP 40 153 et EP 136 214 mais on n'a pu obtenir que des chloroformiates alpha-chlorés.

Il était par conséquent d'un grand intérêt de trouver un nouveau procédé de préparation des carbonates alpha-halogénés qui ne présente pas les inconvénients mentionnés ci-dessus.

Le nouveau procédé selon l'invention consiste à préparer les carbonates alpha-halogénés de formule:

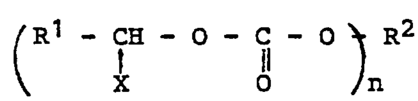

par réaction d'un aldéhyde particulier de formule $R^1CHO$ dans laquelle $R^1$ représente

– un atome d'hydrogène,

– un radical aliphatique en $C_1$ à $C_{12}$, linéaire ou ramifié, saturé ou non, substitué ou non par un ou plusieurs atomes d'halogène,

– un radical cycloaliphatique en $C_3$ à $C_{20}$ saturé ou non, porteur ou non d'un ou de plusieurs substituants choisis parmi les atomes d'halogène et les radicaux hydrocarbonés en $C_1$ à $C_{10}$,

– un radical araliphatique en $C_7$ à $C_{15}$, porteur ou non d'un ou de plusieurs substituants choisis parmi les atomes d'halogène, les radicaux hydrocarbonés en $C_1$ à $C_{10}$ et les groupes alcoxy, aryloxy, aralkyloxy, cyano et trifluorométhyle,

– ou un radical aromatique en $C_6$ à $C_{14}$, porteur ou non d'un ou de plusieurs substituants choisis parmi les atomes d'halogène, les radicaux hydrocarbonés en $C_1$ à $C_{10}$ et les groupes alcoxy, aryloxy, aralkyloxy, cyano et trifluorométhyle,

avec un halogénoformiate particulier de formule

$$R^2 \left( O - \underset{\underset{O}{\|}}{C} - X \right)_n$$

dans laquelle n est égal à 1 ou 2,

$R^2$ représente

– un radical aliphatique en $C_2$ à $C_{20}$, insaturé au moins en position 1, linéaire ou ramifié, substitué ou non par un ou plusieurs atomes d'halogène,

– un radical aromatique en $C_6$ à $C_{14}$ porteur ou non d'un ou plusieurs substituants choisis parmi les atomes d'halogène, les radicaux hydrocarbonés en $C_1$ à $C_{10}$ et les groupes alcoxy, aryloxy, aralkyloxy, cyano et trifluorométhyle,

– un radical aliphatique en $C_1$ à $C_{20}$ et monohalogéné en position 1 qui peut porter un ou plusieurs autres atomes d'halogène en position 2 ou supérieure à 2, saturé on non

– un groupe succinimido,

– ou un groupe trihalogéno-2,2,2 éthyle,

et X représente un atome de fluor, chlore, brome ou iode; ou $R^2$ représente

– un radical aliphatique en $C_1$ à $C_{20}$, linéaire ou ramifié, porteur ou non d'un ou de plusieurs substituants choisis parmi les atomes d'halogène et les radicaux alcoxy, aryloxy, aralkyloxy et aryle,

et X représente un atome de fluor, en présence d'une quantité comprise entre 0,02 et 0,15 équivalent par équivalent molaire d'halogénoformiate, d'un catalyseur choisi dans le groupe qui comprend les amines tertiaires aromatiques, les amines hétéroaromatiques, les pyridones, les guanidines pentaalkylsubstituées, les phosphines tertiaires, les halogénures d'ammonium quaternaire, les halogénures de phosphonium quaternaire, les halogénures alcalins associés à un complexant de leurs cations, les urées tétrasubstituées et leur produit de réaction avec le phosgène, à une température comprise entre 50° et 120°C.

Le schéma réactionnel est le suivant:

$$nR^1CHO + R^2 \left( O - \underset{\underset{O}{\|}}{C} - X \right)_n \xrightarrow{\text{catalyseur}} \left( R^1 - \underset{\underset{X}{|}}{CH} - O - \underset{\underset{O}{\|}}{C} - O \right)_n R^2$$

Ce nouveau procédé est particulièrement avantageux par rapport au procédé antérieur parce que les produits de départ sont des produits disponibles dans le commerce ou faciles à préparer et stables, les quantités de catalyseur utilisées sont très faibles, sa sa mise en oeuvre est simple et les rendements sont excellents. Des carbonates alpha-chlorés et surtout des carbonates bromés, iodés ou fluorés qui n'avaient jamais pu être préparés auparavant, sont obtenus maintenant grâce à celui-ci.

Les aldéhydes de départ qui peuvent être utilisés dans le cadre de l'invention sont numbreux. En particulier on choisit les aldéhydes dont le radical $R^1$ est un atome d'hydrogène, un radical substitué ou non, aliphatique en $C_1$ à $C_8$, linéaire ou ramifié, saturé ou non, cycloaliphatique en $C_3$ à $C_{10}$, saturé ou non, araliphatique en $C_7$ à $C_{13}$ ou aromatique en $C_6$ à $C_{10}$.

Le ou les substituants de $R^1$ sont en particulier choisis dans le groupe qui comprend les atomes d'halogène, les radicaux hydrocarbonés en $C_1$ à $C_6$, les groupes alkoxy en $C_1$ à $C_3$ et les groupes aryloxy en $C_6$ à $C_{10}$.

Comme aldéhydes satisfaisants on peut citer le formaldéhyde, l'acétaldéhyde, le trichloroacétaldéhyde, le propionaldéhyde, le cyclohexanal, le dichloro-1-isopropanal, le benzaldéhyde, le butyraldéhyde.

L'aldéhyde est généralement utilisé en quantité stoechiométrique. Il peut quelquefois être ajouté en léger excès.

Les halogénoformiates particuliers utiles pour la mise en oeuvre du procédé selon l'invention se trouvent dans le commerce ou se préparent facilement par réaction d'un halogénure de carbonyle avec l'alcool ou avec l'aldéhyde correspondant comme décrit dans l'article du Chemical Reviews 64, pages 645-649 (1964) et dans le brevet EP nº 40 153, par réaction du phosgène sur un sel de mercure comme décrit dans les brevets EP nº 2 973, FR nº 2 381 789 et FR nº 2 421 866 ou par réaction d'un halogénoformiate halogéné sur le zinc ou le magnésium comme décrit dans la demande FR nº 8512652. Ils sont très stables dans les conditions réactionnelles du procédé de l'invention, ce qui est très avantageux.

On utilise en particulier les halogénoformiates dont le radical $R^2$ est un radical substitué ou non, aliphatique en $C_2$ à $C_{10}$ et insaturé au moins en position 1, aromatique en $C_6$ à $C_{10}$, aliphatique en $C_1$ à $C_{10}$ et monohalogéné en position 1 et porteur ou non d'un ou de plusieurs atomes d'halogène en position 2 ou supérieure à 2, de préférence l'halogène étant le chlore, saturé ou non, le radical succinimido ou le radical trichloro-2,2,2 éthyle, lorsque X représente un atome de fluor, chlore, brome ou iode, ou un radical aliphatique en $C_1$ à $C_{10}$, linéaire ou ramifié, lorsque X représente un atome de fluor.

Le ou les substituants de $R^2$ sont en particulier choisis dans le groupe qui comprend les atomes d'halogène, de préférence l'atome de chlore, les radicaux hydrocarbonés en $C_1$ à $C_6$ et les groupes aryloxy en $C_6$ à $C_{10}$.

Les halogénoformiates préférés sont ceux dont le radical $R^2$ est un radical vinyle, isopropényle, dichloro-2,2 vinyle, cyclohexényle, chlorométhyle, trichloro-2,2,2 éthyle, tétrachloro-1,2,2,2 éthyle, alpha-chloroéthyle, alpha-chloropropényle, phényle, bisphényle A, lorsque X représente un atome de fluor, de chlore, de brome ou d'iode, ou $R^2$ est un radical éthyle, méthyle, propyle, isopropyle, butyle, isobutyle, octyle, lorsque X est un atome de fluor.

L'halogénoformiate est de préférence introduit en quantité stoechiométrique.

Lorsque les catalyseurs sont du type amines tertiaires on peut utiliser des composés de la famille des pyridines, tel que la pyridine, la diméthylamino-4 pyridine ou la quinoléine, des imidazoles tels que le N-méthylimidazole ou le benzimidazole, des anilines telles que la N,N-diméthylaniline ou la N,N-diéthylaniline, des composés tels que la cétone de Mischler.

Comme exemple d'autres types de catalyseurs on peut citer les pyridones telles que la N-méthyl pyridone, la tributylphosphine, le chlorure de tétra-n-hexylammonium, le chlorure de tripropylbenzylammonium, le chlorure de tétrabutylchloroimmonium, la tétrabutylméthylguanidine, les chlorures de phosphonium quaternaire, les halogénures de sodium et de potassium associés à des complexants tels que ceux décrits dans l'article de Kappenstein, Bull. Soc. Chim. de France nº 1-2, pages 89-109 (1974) ou dans celui de Lehn, Structure and Bonding, Volume 16, pages 2-64, Springer Verlag (1974), en particulier au 18-couronne-6 ou au cryptate (2,2,2).

Les catalyseurs peuvent éventuellement être greffés sur un polymère par exemple sur un polystyrène, pour faciliter leur séparation du milieu réactionnel et leur recyclage.

Les catalyseurs préférés sont choisis dans le groupe qui comprend la pyridine, la méthyl-2 éthyl-5 pyridine, la diméthylamino-4 pyridine, la quinoléine, le N-méthylimidazole, le chlorure de tétra n-hexylammonium, le fluorure ou chlorure de potassium associé au 18-couronne-6, la tributylphosphine, et le chlorure de tributylphosphonium greffé sur un polystyrène.

Les quantités préférées de catalyseur sont comprises entre 0,02 et 0,06 équivalent molaire par équivalent d'halogénoformiate.

La réaction peut être effectué sans solvant mais généralement on préfère utiliser un solvant inerte vis à vis des réactifs. Comme solvants convenant bien on peut citer les solvants aliphatiques chlorés tels que le dichloro-1,2 éthane ou le tétrachlorure de carbone, les solvants aromatiques tels que le toluène, les nitriles tels que l'acétonitrile, les éthers cycliques tels que le tétrahydrofuranne et le dioxanne.

La température de la réaction est de préférence comprise entre 60 et 100°C et la pression est la pression atmosphérique. La durée de la réaction est quant à elle généralement comprise entre 1h et 24h, de préférence entre 1h et 10h.

A la fin de la réaction on récupère le carbonate alpha-halogéné par des méthodes classiques telles qu'une distillation fractionnée ou une cristallisation.

Les rendements en carbonate alpha-halogéné sont très souvent supérieurs à 80%.

Comme on peut le constater le procédé de l'invention permet d'obtenir facilement à partir de composés simples, les carbonates alpha-halogénés avec un très bon rendement et pratiquement sans sous-produits.

Pour sa mise en oeuvre il n'est pas nécessaire d'introduire en grand excès l'un des réactifs, les opérations de purification en sont simplifiées, le rendement amélioré et le procédé est par conséquent très économique.

Les carbonates alpha-halogénés obtenus sont très utiles pour effectuer la synthèse de médicaments tels que des antibiotiques, des antiinflammatoires, des antihypertenseurs ou certains antifibrinolytiques, en particulier pour modifier les fonctions acides carboxyliques, comme décrit dans les brevets FR nº 2 201 870, nº 2 387 988, EP nº 79 872, nº 82 404, DE nº 2 311 328, nº 2 311 664 ou US nº 4 426 391 ou pour former des insecticides comme décrit dans le brevet DE nº 2 628 410.

Les exemples qui suivent illustrent l'invention sans toutefois la limiter.

EXEMPLE 1

Préparation du carbonate de chloro-1-éthyle et de phényle

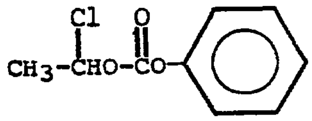

On ajoute 0,2 g (2,5 mmoles) de pyridine à une solution de 8,0 g (50 mmoles) de chloroformiate de phényle et 3,2 g (70 mmoles) d'acétaldéhyde dans 30 ml de dichloro-1,2 éthane et on porte à 80°C pendant 5 heures.

Après refroidissement, on lave avec 50 ml d'eau, on extrait la phase aqueuse avec 50 ml de dichlorométhane, on rassemble les phases organiques, on sèche sur $MgSO_4$ et on distille sous pression réduite.

On recueille 7,3 g (rendement, R = 71 %) du produit attendu.

EXEMPLES 2 à 8

On prépare et on recueille comme à l'exemple 1, d'autres carbonates de chloro-1 alkyle ou aralkyle et d'hydrocarbyle. Les résultats sont rassemblés dans le tableau 1.

TABLEAU 1

| Ex. | Aldéhyde (équivalent) | $R^2$ | Catalyseur (équivalent) | Solvant | Température (°C) | Durée | Rendement[b] % |
|---|---|---|---|---|---|---|---|
| 2 | $Cl_3CCHO$ (1,0) | $CH_3CHCl-$ | Pyridine (0,09) | DCE[a] | 83 | 1 h | 90 |
| 3 | $CH_3CHO$ (1,6) | $CH_3CHCl-$ | Pyridine (0,05) | DCE | 60 | 4 h | 49 |
| 4 | $Cl_3CCHO$ (1,1) | $ClCH_2-$ | Pyridine (0,04) | DCE | 80 | 5 h | 76 |
| 5 | $Cl_3CCHO$ (1,2) | $CH_3CHCl-$ | $Hex_4N^{\oplus}Cl^{\ominus}$ (0,04) | Toluène | 100 | 6 h | 87 |
| 6 | PhCHO (1,1) | $CH_2=C(Me)-$ | Pyridine (0,05) | DCE | 83 | 20 h | 67 |
| 7 | $Cl_3CCHO$ (1,6) | $Cl_3CCHCl-$ | Pyridine (0,06) | DCE | 80 | 4 h | 88 |
| 8 | $Cl_3CCHO$ (1,1) | $CH_2=CHCHCl-$ | $PhCH_2N^{\oplus}Pr_3Cl^{\ominus}$ (0,06) | DCE | 80 | 18 h | 49 |

a : DCE = Dichloro-1,2 éthane b : après distillation

EXEMPLE 9

Préparation du carbonate de chloro-1-éthyle et de tétrachloro-1,2,2,2-éthyle

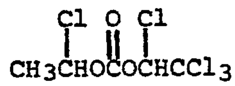

On chauffe à 100°C un mélange de 6,0 g (42 mmoles) de chloroformiate de chloro-1-éthyle, de 6,5 g (44 mmoles) de chloral, 0,5 g (6,7 mmoles) de KCl, de 0,5 g (1,8 mmoles) de 18-couronne-6 et de 1,1 g (7,0 mmoles) d'undécane (comme étalon interne). Après 17 heures, le rendement calculé est 57 % (par CPV). Bien que la réaction ne soit pas encore finie, on distille le produit (Eb = 69-70°C/0,15 mm Hg) et on obtient 6,5 g (R = 53 %) du produit attendu.

EXEMPLES 10 A 17

On prépare le carbonate de chloro-1-éthyle et de tétrachloro-1,2,2,2-éthyle comme à l'exemple 9 mais en présence de différents autres catalyseurs et dans un solvant. Comme à l'exemple 9 les réactions ne sont pas menées à terme. Les rendements ne sont donc pas optimisés. Les résultats sont rassemblés dans le tableau 2.

TABLEAU 2

| Ex. | Equivalents de $Cl_3CCHO$ | Catalyseur (équiv.) | Solvant | Température (C°) | Durée | Rendement[b] (%) |
|---|---|---|---|---|---|---|
| 10 | 1,06 | $PBu_3$ (0,05) | DCE[a] | 83 | 11 h | 72 |
| 11 | 1,07 | $PhNEt_2$ (0,07) | DCE | 83 | 11 h | 36 |
| 12 | 1,07 | 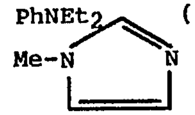 (0,06) | DCE | 83 | 11 h | 66 |
| 13 | 1,10 | (P)$-CH_2\overset{\oplus}{P}Bu_3\ \overset{\ominus}{Cl}$ (0,04)[c] | DCE | 83 | 11 h | 78 |
| 14 | 1,08 | 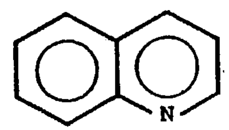 (0,05) | DCE | 83 | 7 h | 89 |
| 15 | 1,07 | $(Bu_2N)_2\overset{\oplus}{C}-Cl\ \overset{\ominus}{Cl}$ (0,03) | DCE | 83 | 17 h | 71 |
| 16 | 1,04 | 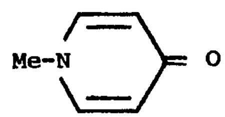 (0,05) | DCE | 83 | 11 h | 78 |
| 17 | 1,05 | 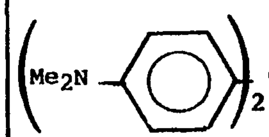 CO (0,06) | DCE | 83 | 11 h | 83 |

a : DCE = dichloro-1,2 éthane

b : rendements calculés par CPV avec de l'undécane comme étalon interne

c : P = polymère (squelette styrènique avec 1 % de divinylbenzène) vendu par la Société Fluka (0,78 mmole $Cl^-/g$).

Exemple 18

Préparation du carbonate de tétrachloro-1,2,2,2-éthyle et de N-oxysuccinimidyle

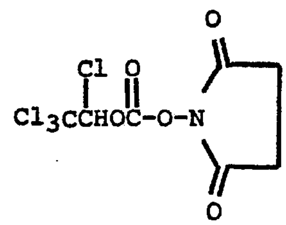

On dissout 1,5 g (0,01 mole) de chloral et 1,8 g (0,01 mole) de chloroformiate de N-oxysuccinimidyle dans 10 ml de dichloro-1,2 éthane. On ajoute 5 gouttes ( 0,2 ml) de pyridine et on chauffe à reflux 1 heure. Après refroidissement, on lave avec de l'acide chlorohydrique 0,1 N puis avec de l'eau. On sèche sur sulfate de magnésium et on évapore pour obtenir 1,3 g du produit attendu (R = 40%).

Exemples 19 à 22

On prépare comme à l'exemple 1 différents carbonates alpha-halogénés. Les résultats sont rassemblés dans le tableau 3.

TABLEAU 3

| Ex. | Aldéhyde (équivalent) | $R_2$- | X | Catalyseur (équivalent) | Solvant | Tempé-rature (°C) | Durée | Rende-ment[c] % |
|---|---|---|---|---|---|---|---|---|
| 19 | 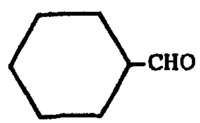-CHO (1,08) | $Cl_3CCH_2$- | Br | 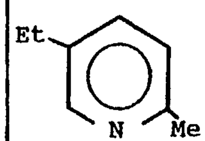 (0,05) | DCE[b] | 83 | 4 h | 37 |
| 20 | $CH_3CHO$ (1,5) | Ph- | Br | Pyridine (0,03) | DCE | 83 | 1 h | 82 |
| 21 | $CH_3CHO$ (1,6) | Ph- | I | Pyridine (0,05) | DCE | 70 | 1,5 h | 80 |
| 22 | $Cl_3CCHO$ (1,06) | Ph- | F | DMAP[a] (0,05) | $CH_3CN$ | 82 | 24 h | 32 |

a : DMAP = N,N-diméthylaminopyridine  b : DCE = dichloro-1,2 éthane

c : Rendement en produit distillé.

Exemple 23

Préparation du carbonate de fluoro-1-chloro-2-méthyl-2-propyle et d'éthyle.

On ajoute du chloro-2-méthyl-2 propanal (4,90 g, 0,046 mole) à un mélange de fluoroformiate d'éthyle (6 g, 0,065 mole) de KF sec (1,70 g, 0,029 mole) et de 18-couronne-6 (1,60 g, 0,0061 mole).

Le mélange est agité à 55°C pendant 1,5 jours. Puis on le refroidit, on le dilue avec $CH_2Cl_2$ (70 ml), on le lave à l'eau (3 x 70 ml), on le sèche ($Na_2SO_4$), on le concentre et on effectue une distillation sous vide pour obtenir 6,95 g (R = 76 %) du carbonate attendu.

Exemple 24

Carbonate de fluoro-1-méthyle et d'octyle

On fait passer du formaldéhyde gazeux préparé à partir de paraformaldéhyde (1,00 g, 0,033 mole) dans le ballon de réaction contenant le fluoroformiate d'octyle (2,40 g, 0,014 mole), du KF sec (1,32 g, 0,014 mole) et du 18-couronne-6 (0,47 g, 0,0018 mole) dans 20 ml d'acétonitrile. Le ballon est maintenu à 65°C pendant la durée de l'addition du formaldéhyde. Après 2 heures, le mélange est dilué avec du $CH_2Cl_2$ (50 ml), lavé avec de l'eau (3 x 50 ml) séché ($Na_2SO_4$), concentré et distillé sous vide pour donner 2,14 g (R = 76 %) du carbonate attendu sous forme d'un liquide incolore.

Exemple 25

Préparation du carbonate de chloro-1-éthyle et de dichloro-2,2 vinyle

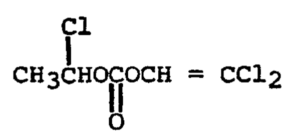

On ajoute 0,35 g (4 mmoles) de pyridine à une solution de 15,5 g (88 mmoles) de chloroformiate de dichloro-2,2 vinyle et 5,6 g (0,13 mole) d'acétaldéhyde dans 70 ml de dichloro-1,2 éthane et on porte à 80°C pendant 1,5 heure. Après refroidissement, on lave le milieu réactionnel à l'eau, on sèche sur $MgSO_4$ et on concentre sous vide.

Après distillation sous pression réduite, on recueille 16,1 g (R = 83%) du produit attendu.

Dans le tableau 4 suivant les propriétés physiques et spectrales des carbonates préparés ci-dessus ont été rassemblées.

TABLEAU 4

| Ex | Carbonate | Point d'ébullition Eb°C/mmHg | RMN $^1$H ppm | IR $cm^{-1}$ | Analyse élémentaire ou spectre de masse |
|---|---|---|---|---|---|
| 1 | 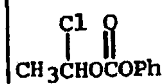 | 67-68/0,15 | 7,5-6,8(m,5H) ; 6,37 (q, J=6, 1H); 1,73(d,J=6,3H) | 1775 | Produit connu |
| 2,5, 9,17 | 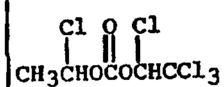 | 69-70/0,15 | 6,64 (s, 1H) ; 6,40 (q, J = 6, 1H) ; 1,86(d,J=6,3H) | 1780 | C 20,85 ; H 1,77 ; Cl 61,47 calculée : C 20,68 ;H 1,74; Cl 61,05 |
| 3 | 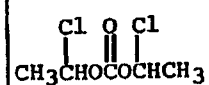 | 67-72/9 | 6,40 et 6,35 (q, J = 6, 2H diastéréomérique) ; 1,80 (d, J = 6, 6H) | 1780 | Produit connu - C 32,36; H 4,28 ; Cl 37,95 calculée : C 32,11 ;H 4,31 ; Cl 37,91 |
| 4 | 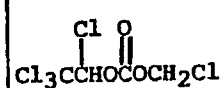 | 60-61/0,11 | 6,60 (s, 1H) ; 5,73 (s,2H) | 1785 | C 17,38 ; H 1,08 ; Cl 64,33 calculée : C 17,39 ; H 1,09 ; Cl 64,15 |

TABLEAU 4 (suite)

| Ex | Carbonate | Point d'ébullition Eb°C/mmHg | RMN $^1H$ ppm | IR $cm^{-1}$ | Analyse élémentaire ou spectre de masse |
|---|---|---|---|---|---|
| 6 | 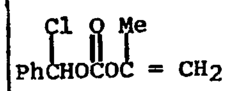 | 92-96/0,2 | 7,7-7,1 (m, 6H) ; 4,78 (s, 1H) ; 4,65 (s, 1H) ; 1,92 (s, 3H) | 1770 1675 | C 58,74 ; H 4,95 ; Cl 15,47 calculée : C 58,29 ; H 4,89 ; Cl 15,64 |
| 7 | 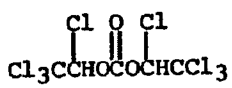 | 90-95/0,04 Point de fusion 63-64°C | 6,67 (s) | 1792 | Produit connu |
| 8 | 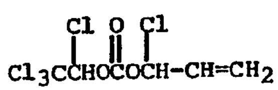 | 74-76/0,03 | 6,72 (d, J = 5, 1H) ; 6,68 (s, 1H) ;6,5-5,2 (m, 3H) | 1782 | C 24,17 ; H 1,69 ; Cl 59,18 calculée :C 23,83 ; H 1,67 ; Cl 58,63 |
| 18 | 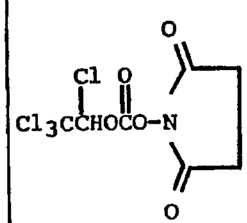 | Point de fusion 108°C | 6,6(s, 1H); 2,90 (s, 4H) | 1760 | Produit connu |

TABLEAU 4 (suite)

| Ex | Carbonate | Point d'ébullition Eb°C/mmHg | RMN $^1H$ ppm | IR $cm^{-1}$ | Analyse élémentaire ou spectre de masse |
|---|---|---|---|---|---|
| 19 | 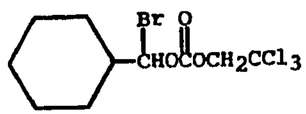 | 117-120/0,04 | 6,35 (d, J = 5, 1H) ; 4,78 (s, 2H) ; 2,5 - 0,8 (m, 11H) | 1770 | C 33,09 ; H 3,84<br>calculée : C 32,60 ; H 3,83 |
| 20 | 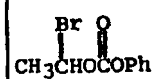 | 74-79/0,03 | 7,6-6,9 (m, 5H) ; 6,56 (q, J = 6, 1H) ; 1,98 (d, J = 6, 3H) | 1775 | C 44,19 ; H 3,77 ; Br 32,88 ; O 19,80<br>calculée : C 44,11 ; H 3,70 ; Br 32,61 ; O 19,58 |
| 21 | 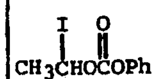 | 93-97/0,45<br>F 59-61°C | 7,6-7,0 (m, 5H) ; 6,75 (q, J = 6, 1 H) ; 2,30 (d, J = 6, 3H) | 1772 | C 37,17 ; H 3,12 ; I 43,23 ;<br>calculée C 37,01 ; H 3,11 ; I 43,45 |
| 22 | 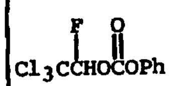 | 82-86/0,03 | 7,6-7,0 (m, 5H) ; 6,43 (d, J = 53) | 1790 | m/e(%) : 290 (1%), 288(2%), 286 (2%) ; 94 (100%), 77(84%), 65 (61%) |

TABLEAU 4 (suite et fin)

| Ex | Carbonate | Point d'ébullition Eb°C/mmHg | RMN $^{1}H$ ppm | IR $cm^{-1}$ | Analyse élémentaire ou spectre de masse |
|---|---|---|---|---|---|
| 23 | 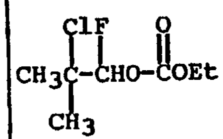 | 60-63/2,5 | 6,04 (d, J = 55, 1H) ; 4,21 (q, J = 7 , 2H) ; 1,57 (s, 6H) ; 1,31 ( t, J = 7, 3H) | 1770 | C 42,55 ; H 6,22 ; calculée C 42,33 ; H 6,09 |
| 24 | 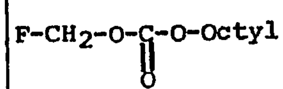 | 82-85/2,5 | 5,58 (d, J = 52, 2H); 4,12 (t, J = 6, 2H) ; 1,30 (m, 14H) ; 0,89 (t, J = 6, 3H) | 1760 | C 58,50 ; H 9,35 ; calculée C 58,23 ; H 9,28 ; |
| 25 | 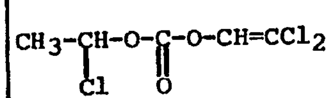 | 44-45/0,1 | 7,46 (s, 1H) ; 6,42 (σ, J = 6, 1H) ; 1,86 (d, J = 6, 3H) | 1782 | C 27,44 ; H 2,36 ; Cl 48,60 ; calculée C 27,37 ; H 2,30 ; Cl 48,47 |

## Revendications

1. Procédé de préparation des carbonates alpha-halogénés de formule

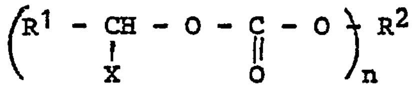

caractérisé en ce que l'on fait réagir un aldéhyde particulier de formule $R^1CHO$ dans laquelle $R^1$ représente
- un atome d'hydrogène,
- un radical aliphatique en $C_1$ à $C_{12}$, linéaire ou ramifié, saturé ou non, substitué ou non par un ou plusieurs atomes d'halogène,
- un radical cycloaliphatique en $C_3$ à $C_{20}$, saturé ou non, porteur ou non d'un ou de plusieurs substituants choisis parmi les atomes d'halogène et les radicaux hydrocarbonés en $C_1$ à $C_{10}$,
- un radical araliphatique en $C_7$ à $C_{15}$, porteur ou non d'un ou de plusieurs substituants choisis parmi les atomes d'halogène, les radicaux hydrocarbonés en $C_1$ à $C_{10}$ et les groupes alcoxy, aryloxy, aralkyloxy, cyano et trifluorométhyle,
- ou un radical aromatique en $C_6$ à $C_{14}$, porteur ou non d'un ou de plusieurs substituants choisis parmi les atomes d'halogène, les radicaux hydrocarbonés en $C_1$ à $C_{10}$ et les groupes alcoxy, aryloxy, aralkyloxy, cyano et trifluorométhyle,

avec un halogénoformiate particulier de formule

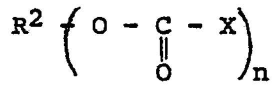

dans laquelle n est égal à 1 ou 2,

$R^2$ représente
- un radical aliphatique en $C_2$ à $C_{20}$, insaturé au moins en position 1, linéaire ou ramifié, substitué ou non par un ou plusieurs atomes d'halogène,
- un radical aromatique en $C_6$ à $C_{14}$ porteur ou non d'un ou plusieurs substituants choisis parmi les atomes d'halogène, les radicaux hydrocarbonés en $C_1$ à $C_{10}$ et les groupes alcoxy, aryloxy, aralkyloxy, cyano et trifluorométhyle,
- un radical aliphatique en $C_1$ à $C_{20}$ et monohalogéné en position 1 qui peut porter un ou plusieurs autres atomes d'halogène en position 2 ou supérieure à 2, saturé ou non
- un groupe succinimido,
- ou un groupe trihalogéno-2,2,2 éthyle,

et X représente un atome de fluor, chlore, brome ou iode;

ou $R^2$ représente
- un radical aliphatique en $C_1$ à $C_{20}$, linéaire ou ramifié, porteur ou non d'un ou de plusieurs substituants choisis parmi les atomes d'halogène et les radicaux alcoxy, aryloxy, aralkyloxy et aryle,

et X représente un atome de fluor, en présence d'une quantité comprise entre 0,02 et 0,15 équivalent par équivalent molaire d'halogénoformiate, d'un catalyseur choisi dans le groupe qui comprend les amines tertiaires aromatiques, les amines hétéroaromatiques, les pyridones, les guanidines pentaalkylsubstituées, les phosphines tertiaires, les halogénures d'ammonium quaternaire, les halogénures de phosphonium quaternaire, les halogénures alcalins associés à un complexant de leurs cations, les urées tétrasubstituées et leur produit de réaction avec le phosgène, à une température comprise entre 50° et 120°C.

2. Procédé de préparation selon la revendication 1 caractérisé en ce que $R^1$ représente un atome d'hydrogène, un radical substitué ou non, aliphatique en $C_1$ à $C_8$, linéaire ou ramifié, saturé ou non, cycloaliphatique en $C_3$ à $C_{10}$, saturé ou non, araliphatique en $C_7$ à $C_{13}$ ou aromatique en $C_6$ à $C_{10}$, le ou les substituants de $R^1$ étant choisis dans le groupe qui comprend les atomes d'halogène, les radicaux hydrocarbonés en $C_1$ à $C_6$, les groupes alkoxy en $C_1$ à $C_3$ et les groupes aryloxy en $C_6$ à $C_{10}$.

3. Procédé de préparation selon la revendication 1 ou 2 caractérisé en ce que $R^2$ représente un radical substitué ou non, aliphatique en $C_2$ à $C_{10}$ et insaturé au moins en position 1, aromatique en $C_6$ à $C_{10}$, aliphatique en $C_1$ à $C_{10}$ et monohalogéné en position 1 et porteur ou non d'un ou de plusieurs atomes d'halogène en position 2 ou supérieure à 2, de préférence l'halogène étant le chlore, saturé ou non, le radical succinimido ou le radical trichloro-2,2,2 éthyle, lorsque X représente un atome de fluor, chlore, brome ou iode, ou un radical aliphatique en $C_1$ à $C_{10}$, linéaire ou ramifié, lorsque X représente un atome de fluor, le ou les substituants de $R^2$ étant choisis dans le groupe qui comprend les atomes d'halogène, de préférence l'atome de chlore, les radicaux hydrocarbonés en $C_1$ à $C_6$ et les groupes aryloxy en $C_6$ à $C_{10}$.

4. Procédé de préparation selon l'une quelconque des revendications précédentes caractérisé en ce que le catalyseur est choisi parmi les composés de la famille des pyridines, des imidazoles, des anilines, des pyridones, des composés tels que la cétone de Mischler, la tributylphosphine, le chlorure de tétra-n-hexylammonium, le chlorure de tripropylbenzylammonium, le chlorure de tétrabutylchloroimmonium, la tétrabutylméthylguanidine, les halogénures de sodium et de potassium associés à un complexant de leur cation.

5. Procédé selon l'une quelconque des revendications précédentes caractérisé en ce que le catalyseur est greffé sur un polymère.

6. Procédé de préparation selon l'une quelconque des revendications précédentes caractérisé en ce que la quantité de catalyseur est comprise entre 0,02 et 0,06 équivalent molaire par équivalent d'halogénoformiate.

7. Procédé de préparation selon l'une quelconque des revendications précédentes caractérisé en ce que la température de la réaction est comprise entre 60° et 100°C.

8. Procédé de préparation selon l'une quelconque des revendications précédentes caractérisé en ce que la réaction est effectuée en présence d'un solvant inerte vis à vis des réactifs.

9. Procédé de préparation selon la revendication précédente caractérisée en ce que le solvant est choisi parmi les solvants aliphatiques chlorés tels que le dichloro-1,2 éthane ou le tétrachlorure de carbone, les solvants aromatiques tels que le toluène, les nitriles tels que l'acétonitrile, les éthers cycliques tels que le tétrahydrofuranne et le dioxanne.

## Claims

1. Process for the preparation of alpha-halogenated carbonates of formula

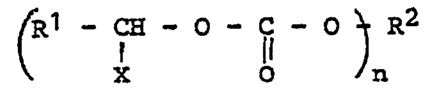

characterized in that a particular aldehyde of formula $R^1CHO$, in which $R^1$ denotes

- a hydrogen atom,
- a saturated or unsaturated, linear or branched, $C_1$–$C_{12}$ aliphatic radical, unsubstituted or substituted by one or more halogen atoms,
- a saturated or unsaturated $C_3$–$C_{20}$ alicyclic radical, unsubstituted or bearing one or more substituents chosen from halogen atoms and $C_1$–$C_{10}$ hydrocarbon radicals,
- a $C_7$–$C_{15}$ araliphatic radical, unsubstituted or bearing one or more substituents chosen from halogen atoms, $C_1$–$C_{10}$ hydrocarbon radicals and alkoxy, aryloxy, aralkoxy, cyano and trifluoromethyl groups, or
- a $C_6$–$C_{14}$ aromatic radical, unsubstituted or bearing one or more substituents chosen from halogen atoms, $C_1$–$C_{10}$ hydrocarbon radicals and alkoxy, aryloxy, aralkoxy, cyano and trifluoromethyl groups,

is reacted with a particular haloformate of formula

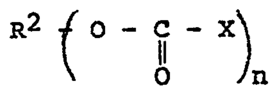

in which n is equal to 1 or 2 $R^2$ denotes

- a linear or branched, $C_2$–$C_{20}$ aliphatic radical unsaturated at least in position 1, unsubstituted or substituted by one or more halogen atoms,
- a $C_6$–$C_{14}$ aromatic radical, unsubstituted or bearing one or more substituents chosen from halogen atoms, $C_1$–$C_{10}$ hydrocarbon radicals and alkoxy, aryloxy, aralkoxy, cyano and trifluoromethyl groups,
- a $C_1$–$C_{20}$ aliphatic radical monohalogenated in position 1, which may bear one or more other halogen atoms in position 2 or a position higher than 2, saturated or unsaturated,
- a succinimido group, or
- a 2,2,2-trihaloethyl group,

and X denotes a fluorine, chlorine, bromine or iodine atom, or $R^2$ denotes

- a linear or branched $C_1$–$C_{20}$ aliphatic radical, unsubstituted or bearing one or more substituents chosen from halogen atoms and alkoxy, aryloxy, aralkoxy and aryl radicals,

and X denotes a fluorine atom, in the presence of a quantity of between 0.02 and 0.15 equivalent per molar equivalent of haloformate, of a catalyst chosen from the group which comprises aromatic tertiary amines, heteroaromatic amines, pyridones, pentaalkylsubstituted guanidines, tertiary phosphines, quaternary ammonium halides, quaternary phosphoniuin halides, alkali metal halides associated with a complexant for their cations, and tetrasubstituted ureas and their reaction product with phosgene, at a temperature of between 50° and 120°C.

2. Process of preparation according to Claim 1, characterized in that $R^1$ denotes a hydrogen atom or a substituted or unsubstituted radical which is a saturated or unsaturated, linear or branched, $C_1$–$C_8$ aliphatic radical, a saturated or unsaturated $C_3$–$C_{10}$ alicyclic radical, a $C_7$–$C_{13}$ araliphatic radical or a $C_6$–$C_{10}$ aromatic radical, the substituent(s) of $R^1$ being chosen from the group which comprises halogen atoms, $C_1$–$C_6$ hydrocarbon radicals, $C_1$–$C_3$ alkoxy groups and $C_6$–$C_{10}$ aryloxy groups.

3. Process of preparation according to Claim 1 or 2, characterized in that $R^2$ denotes a substituted or unsubstituted radical which is a $C_2$–$C_{10}$ aliphatic radical unsaturated at least in position 1, a $C_6$–$C_{10}$ aromatic radical, a $C_1$–$C_{10}$ aliphatic radical monohalogenated in position 1 and bearing or not bearing one or more halogen atoms in position 2 or a position higher than 2, the halogen being preferably chlorine, saturated or unsaturated, the succinimido radical or the 2,2,2-trichloroethyl radical, when X denotes a fluorine, chlorine, bromine or iodine atom, or a linear or branched $C_1$–$C_{10}$ aliphatic radical, when X denotes a fluorine atom, the substituent(s) in $R^2$ being chosen from the group which comprises halogen atoms, preferably the chlorine atom, $C_1$–$C_6$ hydrocarbon radicals and $C_6$–$C_{10}$ aryloxy groups.

4. Process of preparation according to any one of the preceding claims, characterized in that the catalyst is chosen from compounds of the group of pyridines, imidazoles, anilines, pyridones, compounds such as Michler's ketone, tributylphosphine, tetra-n-hexylammonium chloride, tripropylbenzylammonium chloride, tetrabutylchloroimmonium chloride, tetrabutylmethylguanidine, and sodium and potassium halides associated with a complexant for their cation.

5. Process according to any one of the preceding claims, characterized in that the catalyst is grafted onto a polymer.

6. Process of preparation according to any one of the preceding claims, characterized in that the quantity of catalyst is between 0.02 and 0.06 molar equivalent per equivalent of haloformate.

7. Process of preparation according to any one of the preceding claims, characterized in that the reaction temperature is between 60° and 100°C.

8. Process of preparation according to any one of the preceding claims, characterized in that the reaction is carried out in the presence of a solvent which is inert towards the reactants.

9. Process of preparation according to the preceding claim, characterized in that the solvent is chosen from chlorinated aliphatic solvents such as 1,2-dichloroethane or carbon tetrachloride, aromatic solvents such as toluene, nitriles such as acetonitrile, and cyclic ethers such as tetrahydrofuran and dioxane.

**Patentansprüche**

1. Verfahren zur Herstellung α-halogenierter Carbonate der Formel

$$\left( R^1 - \underset{\substack{| \\ X}}{CH} - O - \underset{\substack{\| \\ O}}{C} - O \right)_n R^2$$

gekennzeichnet durch Umsetzung eines Aldehyds der Formel $R^1CHO$, in der $R^1$ bedeutet:
- ein Wasserstoffatom,
- eine geradkettige oder verzweigte, gesättigte oder ungesättigte und ggfs. mit einem oder mehreren Halogenatomen substituierte aliphatische $C_{1-12}$-Gruppe,
- eine gesättigte oder ungesättigte cycloaliphatische $C_{3-20}$-Gruppe, die ggfs. einen oder mehrere Substituenten trägt, die unter Halogenatomen und $C_{1-10}$-Kohlenwasserstoffgruppen ausgewählt sind,
- eine araliphatische $C_{7-15}$-Gruppe, die ggfs. einen oder mehrere Substituenten trägt, die unter Halogenatomen, $C_{1-10}$-Kohlenwasserstoffgruppen sowie Alkoxy, Aryloxy, Aralkyloxy, Cyano und Trifluormethyl ausgewählt sind,

oder
- eine aromatische $C_{6-14}$-Gruppe, die ggfs. einen oder mehrere Substituenten trägt, die unter Halogenatomen, $C_{1-10}$-Kohlenwasserstoffgruppen sowie Alkoxy, Aryloxy, Aralkyloxy, Cyano und Trifluormethyl ausgewählt sind, mit einem Halogenformiat der Formel

$$R^2 \left( O - \underset{\substack{\| \\ O}}{C} - X \right)_n$$

in der bedeuten:

n 1 oder 2 und entweder

R2
- eine zumindest in 1-Stellung ungesättigte geradkettige oder verzweigte und ggfs. mit einem oder mehreren Halogenatomen substituierte aliphatische $C_{2-20}$-Gruppe,
- eine aromatische $C_{6-14}$-Gruppe, die ggfs. einen oder mehrere Substituenten trägt, die unter Halogenatomen, $C_{1-10}$-Kohlenwasserstoffgruppen sowie Alkoxy, Aryloxy, Aralkyloxy, Cyano und Trifluormethyl ausgewählt sind,

– eine gesättigte oder ungesättigte und in 1-Stellung monohalogenierte aliphatische $C_{1-20}$-Gruppe, die ggfs. ein oder mehrere Halogenatome in 2-Stellung oder einer Stellung mit höherer Indizierung trägt,
– eine Succinimidogruppe
oder
– eine 2,2,2-Trihalogenethylgruppe
und
X Fluor, Chlor, Brom oder Jod
oder
$R^2$ eine geradkettige oder verzweigte aliphatische $C_{1-20}$-Gruppe, die ggfs. einen oder mehrere Substituenten trägt, die unter Halogenatomen und Alkoxy, Aryloxy, Aralkyloxy und Aryl ausgewählt sind,
und
X Fluor,
in Gegenwart eines Katalysators in einer Menge von 0,02 bis 0,13 Moläquivalent pro Moläquivalent Halogenformiat, der ausgewählt ist unter aromatischen tertiären Aminen, heteroaromatischen Aminen, Pyridonen, pentaalkylsubstituierten Guanidinen, tertiären Phosphinen, quaternären Ammoniumhalogeniden, quaternären Phosphoniumhalogeniden, mit einem Komplexbildner ihrer Kationen assoziierten Alkalihalogeniden, tetrasubstituierten Harnstoffen und ihren Reaktionsprodukten mit Phosgen, bei einer Temperatur von 50 bis 120°C.

2. Verfahren nach Anspruch 1, dadurch gekennzeichnet, daß $R^1$ ein Wasserstoffatom, eine geradkettige oder verzweigte, gesättigte oder ungesättigte aliphatische $C_{1-8}$-Gruppe, eine gesättigte oder ungesättigte cycloaliphatische $C_{3-10}$-Gruppe, eine araliphatische $C_{7-13}$-Gruppe oder eine aromatische $C_{6-10}$-Gruppe bedeutet, wobei diese Gruppen $R^1$ gegebenenfalls mit einem oder mehreren unter Halogenatomen, $C_{1-6}$-Kohlenwasserstoffgruppen, $C_{1-3}$-Alkoxygruppen und $C_{6-10}$-Aryloxygruppen ausgewählten Substituenten substituiert sind.

3. Verfahren nach Anspruch 1 oder 2, dadurch gekennzeichnet, daß $R^2$ darstellt:
Eine zumindest in 1-Stellung ungesättigte aliphatische $C_{2-10}$-Gruppe, eine aromatische $C_{6-10}$-Gruppe, eine gesättigte oder ungesättigte und in 1-Stellung monohalogenierte aliphatische $C_{1-10}$-Gruppe, die ggfs. ein oder mehrere Halogenatome in 2-Stellung oder in einer Stellung mit höherer Indizierung trägt, wobei das Halogen vorzugsweise Chlor ist, Succinimido oder 2,2,2-Trichlorethyl, wenn X Fluor, Chlor, Brom oder Jod bedeutet, oder eine geradkettige oder verzweigte aliphatische $C_{1-10}$-Gruppe, wenn X Fluor bedeutet, wobei diese Gruppen $R^2$ gegebenenfalls mit einem oder mehreren Halogenatomen, vorzugsweise Chlor, $C_{1-6}$-Kohlenwasserstoffgruppen und $C_{6-10}$-Aryloxygruppen ausgewählten Substituenten substituiert sind.

4. Verfahren nach einem der Ansprüche 1 bis 3, dadurch gekennzeichnet, daß der Katalysator ausgewählt wird unter Verbindungen aus der Familie der Pyridine, Imidazole, Aniline, Pyridone, Verbindungen wie Michlers Keton, Tributylphosphin, Tetra-n-hexylammoniumchlorid, Tripropylbenzylammoniumchlorid, Tetrabutylchlorammoniumchlorid, Tetrabutylmethylguanidin sowie Natrium- und Kaliumhalogeniden, die mit einem Komplexbildner ihrer Kationen assoziiert sind.

5. Verfahren nach einem der Ansprüche 1 bis 4, dadurch gekennzeichnet, daß der Katalysator auf einem Polymer aufgepfropft ist.

6. Verfahren nach einem der Ansprüche 1 bis 5, dadurch gekennzeichnet, daß die Menge des Katalysators 0,02 bis 0,06 Moläquivalent pro Moläquivalent Halogenformiat beträgt.

7. Verfahren nach einem der Ansprüche 1 bis 6, dadurch gekennzeichnet, daß die Reaktionstemperatur 60 bis 100°C beträgt.

8. Verfahren nach einem der Ansprüche 1 bis 7, dadurch gekennzeichnet, daß die Reaktion in Gegenwart eines gegenüber den Reaktanten inerten Lösungsmittels durchgeführt wird.

9. Verfahren nach Anspruch 8, dadurch gekennzeichnet, daß das Lösungsmittel unter aliphatischen chlorierten Lösungsmitteln wie 1,2-Dichlorethan oder Tetrachlorkohlenstoff, aromatischen Lösungsmitteln wie Toluol, Nitrilen wie Acetonitril und cyclischen Ethern wie Tetrahydrofuran und Dioxan ausgewählt wird.